# EUROPEAN PATENT APPLICATION

(11) **EP 2 517 749 A1**
(43) Date of publication of application: **31.10.2012**
(21) Application number: 12155201.2
(22) Date of filing: 13.02.2012
(51) Int. Cl.: A61M 25/00

(54) **Catheter**

(30) Priority: 25.04.2011 JP 2011096941
(71) Applicant: Asahi Intecc Co., Ltd., Nagoya-shi, Aichi 463-0024 (JP)
(72) Inventor: Katsurada, Takeharu, Nagoya-shi, Aichi 463-0024 (JP); Kitagawa, Masanori, Naogya-shi, Aichi 463-0024 (JP)
(74) Representative: Winter, Brandl, Fürniss, Hübner, Röss, Kaiser, Polte - Partnerschaft

(57) **Abstract**

An object of the present invention is to provide a catheter (10) capable of improving torque transmissibility by arranging a reinforcing member (56) on a shaft (50) and of preventing the front end of the catheter (10) from being stuck and caught in a stenosis or a stent strut. The catheter (10) includes: an inner shaft (50) including a

tubular inner layer (54), a braid (56) arranged on the inner layer (54), and an outer layer (58) made of resin and covering the braid (56); and a tubular tip (60) attached to a front end of the inner shaft (50), where the tip (60) has a portion thicker than the front end of the inner shaft (50), and further has an outer diameter decreasing toward a front end thereof.

## Description

### Cross Reference to Related Applications

This application claims priority from Japanese Patent Application No. 2011-096941 filed with the Japan Patent Office on April 25, 2011, the entire content of which is hereby incorporated by reference.

### Technical Field

The present invention relates to a catheter to be inserted into, for example, a stenosis in a body cavity such as a vessel.

### Background Art

Examples of the catheter in the related art, which is inserted into a stenosis in a body cavity such as a vessel for treatment, include a balloon catheter used to dilate the stenosis. The balloon catheter mainly includes a balloon to be dilated, an outer shaft, and an inner shaft arranged in the outer shaft. The inner shaft is for inserting a guidewire therethrough. The outer shaft is used to flow a liquid, such as a contrast agent or saline for dilating the balloon, into a lumen provided between the outer shaft and the inner shaft.

Such a balloon catheter is inserted into a vessel or the like and positioned at a predetermined site. For the insertion and positioning, an operator such as a physician manipulates the balloon catheter by transmitting torque from a proximal side to a front end of the balloon catheter. The transmitted torque is mainly a so-called pushing force for pushing the catheter in the axial direction. The balloon catheter needs to have a high capability to transmit the pushing force from the proximal side to the front side thereof (pushability).

In the related art, there has been proposed a balloon catheter with a reinforcing member such as a braid arranged in a shaft in order to improve the pushability (refer to, for example, JP-A-1-121067, JP-A-2001-157712, and JP-A-2010-115375.

In an attempt to pass a catheter such as a balloon catheter through a relatively severe stenosis or between stent struts of a stent arranged in advance, it may become difficult for the catheter to pass through when the front end thereof is stuck and caught in the stenosis or stent strut. However, such a situation is not sufficiently resolved by the improvement of the pushability achieved by arranging the braid in the shaft as in the balloon catheter in the related art. Therefore, a further improvement is required.

### Summary of Invention

The present invention has been made in view of the above circumstances. An object of the present invention is to provide a catheter capable of improving torque transmissibility by arranging a reinforcing member on a shaft and of preventing the front end of the catheter from being stuck and caught in a stenosis or a stent strut.

In the present invention, the above object can be achieved by a catheter according to claim 1. Preferred embodiments thereof are subject-matter of dependent claims.

A catheter according to the present invention includes: a shaft including a tubular inner layer, a reinforcing member arranged on an outer circumferential surface of the inner layer, and an outer layer covering the reinforcing member; and a tubular tip made of resin and attached to a front end of the shaft, where the tip has a portion thicker than the front end of the shaft, and the tip has an outer diameter decreasing toward a front end of the tip.

### Brief Description of Drawings

The foregoing and other objects, features, aspects and advantages of the invention will become more apparent from the following detailed description when taken in conjunction with the accompanying drawings.
Fig. 1 is a view illustrating an entire balloon catheter according to the present embodiment;
Fig. 2 is an enlarged view of portion A in Fig. 1;
Fig. 3 is a cross-sectional view seen in the III-III direction in Fig. 2;
Fig. 4 is an enlarged view of portion B in Fig. 1;
Fig. 5 is a cross-sectional view of an inner shaft according to the present embodiment;
Fig. 6 is a view illustrating a braid according to the present embodiment;
Fig. 7 is a view illustrating another embodiment;
Fig. 8 is a view illustrating still another embodiment; and
Fig. 9 is a view illustrating a catheter according to another embodiment.

### Description of Embodiments

Preferred embodiments of the present invention are described below with reference to the accompanying drawings, in which like reference characters designate similar or identical parts throughout the several views thereof.
<1> A catheter according to a first aspect of the present invention includes: a shaft including a tubular inner layer, a reinforcing member arranged on an outer circumferential surface of the inner layer, and an outer layer covering the reinforcing member; and a tubular tip made of resin and attached to a front end of the shaft, where the tip has a portion thicker than the front end of the shaft, and the tip has an outer diameter decreasing toward a front end of the tip.
<2> A second aspect of the present invention is the catheter according to the first aspect, wherein the tip has an inner diameter smaller than that of the shaft to make the portion of the tip thicker than the front end of the shaft.
<3> A third aspect of the present invention is the catheter according to the first aspect, wherein the portion of the tip thicker than the front end of the shaft is positioned at a rear end of the tip.
<4> A fourth aspect of the present invention is the catheter according to the first aspect, further including: a balloon; and a tubular outer shaft to which at least part of the balloon is joined, wherein the shaft is inserted into the outer shaft.

<1> In the catheter according to the first aspect of the present invention, the reinforcing member is arranged on the shaft. Therefore, the torque such as pushing force or rotational force applied from the rear side of the catheter to the shaft can be effectively transmitted from the rear side to the front side of the shaft. At the connecting portion between the front end of the shaft and the tip, the thickness of the tip changes in accordance with the absence of the reinforcing member, that is, the tip has a larger thickness than the front end of the shaft. This configuration can prevent the sudden stiffness change caused by the absence of the reinforcing member. Therefore, the torque such as pushing force or rotational force can be transmitted without being blocked at the portion where the stiffness change occurs. As a result, the torque can be effectively transmitted to the tip that is the front end portion of the catheter. Therefore, even when the front end of the catheter is stuck and caught in a stenosis or a stent strut, such a situation can easily be resolved by, for example, rotating the front end of the catheter.
<2> In the second aspect of the present invention, the tip has a smaller inner diameter than the shaft. Therefore, the tip has the portion thicker than the front end of the shaft. With this configuration, the tip is flexibly bent along the guidewire when the catheter passes through a tortuous vessel or a stenosis, or between stent struts, for example, along the guidewire. Furthermore, a stepped portion is hardly generated between the front end of the tip and the guidewire. Therefore, the catheter can smoothly pass through the tortuous vessel or stenosis, or between the stent struts, for example.
<3> In the third aspect of the present invention, the portion thicker than the front end of the shaft is positioned at the rear end of the tip. That is, a sudden stiffness change can be prevented as far as possible from occurring between the front end of the shaft with the reinforcing member provided thereon and the rear end of the tip without the reinforcing member. The outer diameter of the tip progressively decreases from the rear end toward the front end of the tip. Therefore, the torque such as pushing force or rotational force can be effectively transmitted from the shaft to the front end of the tip.
<4> In the fourth aspect of the present invention, the shaft of the first aspect is used as the inner shaft accommodated in the outer shaft of the balloon catheter. With this configuration, the torque such as pushing force or rotational force can be effectively transmitted to the front end of the inner shaft. Therefore, the torque is transmitted without being blocked by the outer shaft. Furthermore, even when the front end of the balloon catheter is stuck and caught in a stenosis or a stent strut while the catheter advances along the guidewire, such a situation can easily be resolved by rotating the tip.

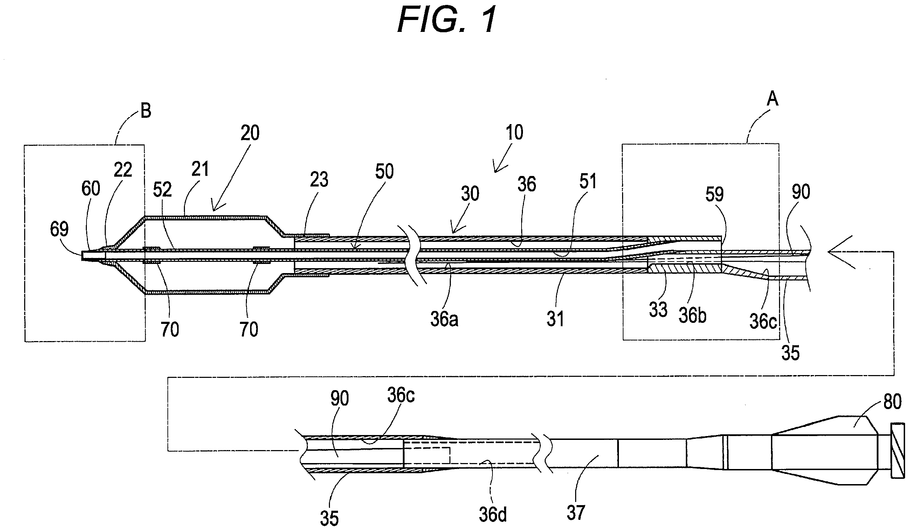

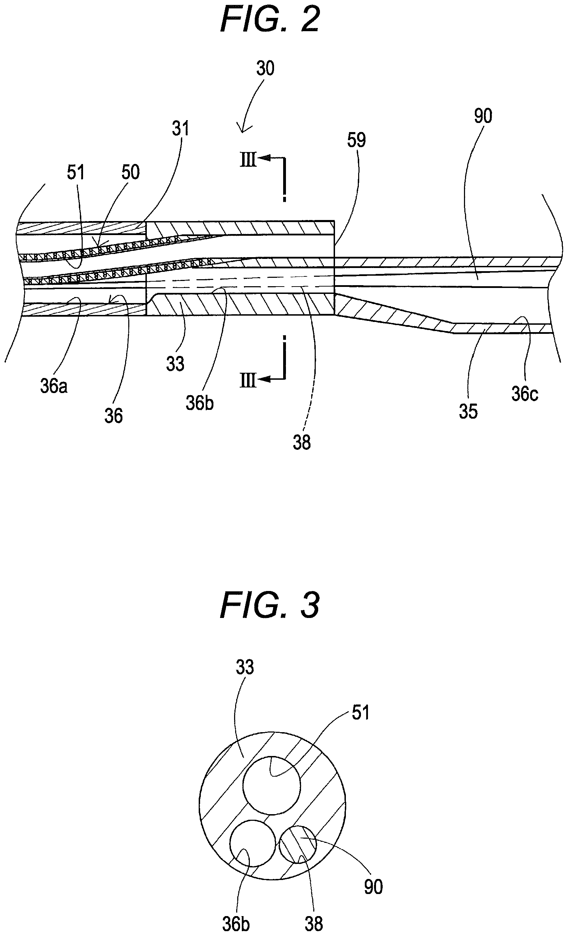

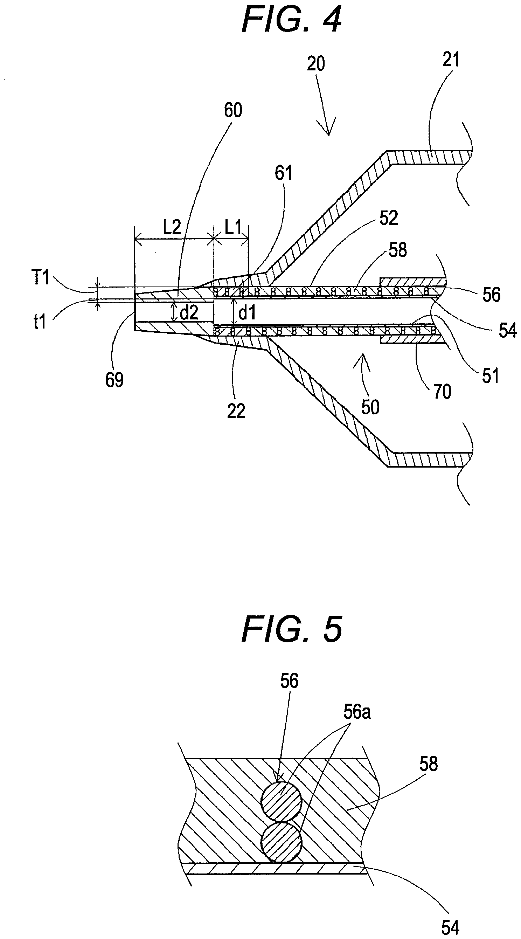

An example in which the catheter according to the present embodiment is applied to a balloon catheter will be described with reference to Figs. 1 to 6. The left side of Fig. 1 illustrates the front side (distal side) to be inserted into the body, and the right side thereof is the rear side (proximal side, base end side) to be manipulated by an operator such as a physician. In Figs. 2 and 4, the front side is illustrated on the left side and the rear side on the right side.

Note that, for easy understanding, the members of the balloon catheter are illustrated in an exaggerated manner in the drawings compared to the actual dimensions.

A balloon catheter 10 is used for the treatment of, for example, an occlusion or a stenosis in a vessel in the heart. The entire length of the balloon catheter 10 is about 1500 mm.

The balloon catheter 10 mainly includes a balloon 20, an outer shaft 30, an inner shaft 50, a tip 60, and a connector 80.

The balloon 20 is made of resin. The balloon 20 includes, in the center in the axial direction thereof, a dilation pan 21 for dilating the balloon 20. The balloon 20 further includes a front end attachment part 22 on the front side of the balloon 20 and a rear end attachment part 23 on the rear side of the balloon 20.

The front end attachment part 22 is bonded to a later-mentioned front end portion (including the tip 60) of an extension part 52 of the inner shaft 50.

The rear end attachment part 23 is bonded to the outer circumferential surface of the front end portion of the outer shaft 30.

The outer shaft 30 is a tubular member constituting an inflation lumen 36 through which the liquid for dilating the balloon 20 is supplied. The outer shaft 30 includes a front outer shaft portion 31, a joint portion 33, a middle outer shaft portion 35, and a rear outer shaft portion 37 in that order from the distal side. The front outer shaft portion 31 and the middle outer shaft portion 35 are tubes made of resin. The joint portion 33 is a portion at which the front outer shaft portion 31, the middle outer shaft portion 35, and the inner shaft 50 are joined by welding. Examples of the resin constituting the front outer shaft portion 31 and the middle outer shaft portion 35 include polyamide, polyamide elastomer, polyolefin, polyester, and polyester elastomer.

The rear end attachment part 23 of the balloon 20 is bonded to the outer circumferential surface of the front end portion of the front outer shaft portion 31.

The front outer shaft portion 31 accommodates the inner shaft 50 therein. A front inflation lumen 36a constituting the front portion of the inflation lumen 36 is formed between the front outer shaft portion 31 and the inner shaft 50.

The front outer shaft portion 31 has substantially the same outer diameter as the joint portion 33. In the present embodiment, the outer diameter of the front outer shaft portion 31 is in the range of about 0.85 mm to about 0.95 mm, and is set at, for example, about 0.90 mm. In the present embodiment, the inner diameter of the front outer shaft portion 31 is in the range of about 0.69 mm to about 0.80 mm, and is set at, for example, about 0.75 mm.

The joint portion 33 joins the front outer shaft portion 31 and the middle outer shaft portion 35. In addition, the joint portion 33 is for attaching the rear end of the inner shaft 50 to the outer shaft 30 in order to form a rear-side guidewire port 59. These members are joined to one another by welding. At the joint portion 33, therefore, the resins constituting the respective members are melted and mixed at the time of welding. A core wire 90 described later is joined to the outer shaft 30 and the inner shaft 50 by welding at the joint portion 33.

In the present embodiment, the axial length of the joint portion 33 is in the range of about 3.0 mm to about 7.0 mm, and is set at, for example, about 5.0 mm.

As shown in Fig. 3, the joint portion 33 includes a communication hole 36b and a core fixing portion 38.

The communication hole 36b passes through the joint portion 33 having a substantially circular cross section. That is, an opening on the front side of the communication hole 36b is in communication with the front inflation lumen 36a. An opening on the rear side of the communication hole 36b is in communication with a middle inflation lumen 36c of the middle outer shaft portion 35.

The core wire 90 described later is inserted into the core fixing portion 38. At the core fixing portion 38, the core wire 90 is fixed to the outer shaft 30 and the inner shaft 50 with the resin constituting the joint portion 33.

The communication hole 36b and the core fixing portion 38 are formed at the time of welding the front outer shaft portion 31, the middle outer shaft portion 35, and the inner shaft 50 to one another. That is, two mandrels respectively used to form the core wire 90 and the communication hole 36b are inserted between the front outer shaft portion 31 and the middle outer shaft portion 35. After that, the front outer shaft portion 31, the middle outer shaft portion 35, and the inner shaft 50 are welded to one another. As a result, one mandrel for forming the core wire 90 is bonded to the joint portion 33 at the core fixing portion 38. The other mandrel for forming the communication hole 36b is pulled out to thereby form the communication hole 36b in the joint portion 33.

The diameter D of the communication hole 36b is set in the range of about 0.20 mm to about 0.30 mm. In the present embodiment, the diameter D is about 0.25 mm.

The middle outer shaft portion 35 is a tubular member made of resin. The middle outer shaft portion 35 includes the middle inflation lumen 36c. The middle inflation lumen 36c is in communication with the communication hole 36b of the joint portion 33, thereby constituting part of the inflation lumen 36.

The middle inflation lumen 36c includes, at the front end portion thereof, a tapered tubular path connected to the communication hole 36b. Except for this tapered tubular path, the middle inflation lumen 36c is a tubular path having a constant outer diameter.

In the present embodiment, the axial length of the middle outer shaft portion 35 is in the range of about 150.0 mm to about 200.0 mm, and is set at, for example, about 160.0 mm. In the present embodiment, the outer diameter of the middle outer shaft portion 35 at the portion with the constant outer diameter is in the range of about 0.80 mm to about 0.90 mm, and is set at, for example, about 0.85 mm. In the present embodiment, the inner diameter of the middle outer shaft portion 35 at the portion with the constant outer diameter is in the range of about 0.65 mm to about 0.80 mm, and is set at, for example, about 0.75 mm.

The rear outer shaft portion 37 is a metal tubular member called hypotube. The front end portion of the rear outer shaft portion 37 is inserted into and bonded to the rear end portion of the middle outer shaft portion 35. A rear inflation lumen 36d formed in the rear outer shaft portion 37 constitutes the inflation lumen 36 together with the front inflation lumen 36a, the communication hole 36b, and the middle inflation lumen 36c described above.

The connector 80 is attached to the rear end of the rear outer shaft portion 37. The liquid such as contrast agent or saline, supplied for dilating the balloon 20 from an indeflator (not shown) attached to the connector 80, passes through the inflation lumen 36 to dilate the balloon 20.

In the present embodiment, the outer diameter of the rear outer shaft portion 37 is in the range of about 0.60 mm to about 0.65 mm, and is set at, for example, about 0.64 mm. The inner diameter of the rear outer shaft portion 37 is in the range of about 0.40 mm to about 0.50 mm, and is set at, for example, about 0.48 mm. The material for the rear outer shaft portion 37 is not particularly limited. In the present embodiment, stainless steel is used as the material. Other materials to be used include a super elastic alloy such as an Ni-Ti alloy.

The core wire 90 is attached to the inner circumferential surface of the front end portion of the rear outer shaft portion 37.

The core wire 90 has a circular cross section. The core wire 90 is a tapered wire made of metal with the diameter progressively decreasing toward the front end thereof. In the present embodiment, the diameter of the core wire 90 progressively decreases toward the distal side gradually from about 0.40 mm to about 0.10 mm.

The material for the core wire 90 is not particularly limited. In the present embodiment, stainless steel (SUS304) is used as the material. Other materials to be used include a piano wire and a super elastic alloy such as an Ni-Ti alloy.

The rear end of the core wire 90 is bonded to the inner wall of the front end portion of the rear outer shaft portion 37 by, for example, brazing or laser welding.

The core wire 90 passes through the middle outer shaft portion 35 and the joint portion 33 and extends to the front end portion of the front outer shaft portion 31. A portion of the core wire 90 on the front side of the portion fixed with the joint portion 33 is a free end. This free end imparts, to the balloon catheter 10, stiffness change in the axial direction.

In the present embodiment, the axial length of the portion of the core wire 90 on the front side of the core fixing portion 38 is preferably in the range of about 5.0 mm to about 150.0 mm, and may be set at, for example, about 130.0 mm.

The core wire 90 is fixed to the front outer shaft portion 31 and the inner shaft 50 with the core fixing portion 38 of the joint portion 33. With this configuration, the core fixing portion 38 transmits the pushing force or rotational force, applied to the core wire 90, to the front outer shaft portion 31 and the inner shaft 5 0 via the joint portion 33.

Note that the pushing force is the force applied by an operator such as a physician for pushing the balloon catheter 10 from the rear side toward the front side thereof in order to advance the balloon catheter into the body. The rotational force is the force applied by the operator for rotating the rear outer shaft portion 37 of the balloon catheter 10 by predetermined degrees around the axis. At this time, the rear outer shaft portion 37 does not necessarily rotate by 360 degrees or more. In other words, the rear outer shaft portion 37 may rotate by less than 360 degrees.

The inner shaft 50 is a tubular member substantially coaxially accommodated in the front outer shaft portion 31. The front inflation lumen 36a is formed between the inner circumferential surface of the front outer shaft portion 31 and the outer circumferential surface of the inner shaft 50. The front inflation lumen 36a constitutes the front end portion of the inflation lumen 36.

The rear end of the inner shaft 50 is welded to the joint portion 33 of the outer shaft 30. As a result, the rear-side guidewire port 59 is formed.

In the present embodiment, the outer diameter of the inner shaft 50 is in the range of about 0.50 mm to about 0.60 mm, and is set at, for example, about 0.53 mm. In the present embodiment, the inner diameter of the inner shaft 50 is in the range of about 0.36 mm to about 0.45 mm, and is set at, for example, about 0.41 mm.

As shown in Figs. 4 to 6, the inner shaft 50 includes an inner layer 54, a braid 56 as a reinforcing member, and an outer layer 58 in that order from the inside in the radial direction.

The inner layer 54 is a tubular member made of resin. The inner layer 54 includes a guidewire lumen 51 through which the guidewire is inserted. The resin material forming the inner layer 54 is not particularly limited. In the present embodiment, polytetrafluoroethylene (PTFE) is used as the material.

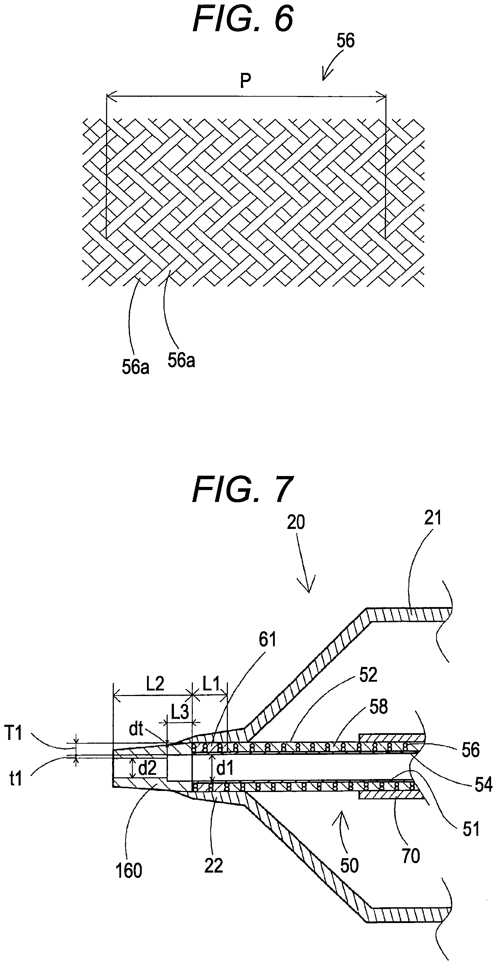

The braid 56 as a reinforcing member is arranged on the surface of the inner layer 54. As shown in Fig. 6, the braid 56 is in the form of a mesh 56a obtained by weaving a plurality of strands. In the present embodiment, every two of eight strands in one direction are woven alternately over and under every two of eight strands in the other direction to weave 16 strands in total into a mesh (8 × 8 strand type). In the present embodiment, the length P (one pitch) shown in Fig. 6 necessary for one strand to go around the surface of the inner layer 54 once is preferably in the range of about 1.0 mm to about 1.5 mm, and may be set at, for example, about 1.3 mm.

Note that the combination of the numbers of strands in the braid is not limited to 16 strands (8 x 8 strand type). For example, 8 strands (4 x 4 strand type) or 4 strands (2 x 2 strand type) may be arranged. Also note that the numbers in the combination may not be the same, and different numbers may be combined, such as a combination of "4 strands x 8 strands" and a combination of "2 strands x 4 strands".

The strand 56a has a circular cross section. The strands 56a wound in the two directions have substantially the same diameter. In the present embodiment, the diameter of the strand 56a is about 0.023 mm. As described above, in the present embodiment, the strands wound in the two directions have substantially the same diameter. The strands 56a wound in the two directions, however, may have different diameters from each other. The cross-sectional shape of the strand is not particularly limited to the circular shape. The cross-sectional shape may be, for example, substantially rectangular or elliptical.

The material for the strand 56a is not particularly limited. Metal is used as this material, for example. In the present embodiment, the material is tungsten which is a radiopaque metal. The material may be resin or other metals such as stainless steel.

The surface of the braid 56 is covered with the outer layer 58 made of resin. The resin material forming the outer layer 58 is not particularly limited, either. Examples of the resin material to be used include polyamide, polyamide elastomer, polyester, and polyurethane. In the present embodiment, polyamide elastomer is used.

The front end portion of the inner shaft 50 includes the extension part 52 extending from the front end of the front outer shaft portion 31. A pair of radiopaque markers 70, spaced apart from each other at a predetermined interval, is attached to a portion of the extension part 52 of the inner shaft 50 in the dilation part 21 of the balloon 20.

The tip 60 is attached to the front end of the extension part 52 of the inner shaft 50. The tip 60 has a tapered outer shape with the outer diameter gradually decreasing toward the front end thereof, and is made of a flexible resin. The resin forming the tip 60 is not particularly limited. Examples of the resin include polyurethane and polyurethane elastomer. In the present embodiment, the resin is polyurethane.

The tip 60 is a cylindrical member constituting the front end portion of the guidewire lumen 51. The tip 60 has a front-side guidewire port 69 at the front end of the tip 60.

The tip 60 is attached to the front end of the inner shaft 50 by welding. For this purpose, a tip attachment part 61 is formed at the boundary between the inner shaft 50 and the tip 60.

The axial length L1 of the tip attachment part 61 (in the rearward direction from the front end of the inner shaft 50) is set at about 1.0 mm. The tip attachment part 61 is formed by covering the front end of the extension part 52 of the inner shaft 50 with the resin constituting the rear end portion of the tip 60, and welding the resin of the rear end portion of the tip 60 and the front end of the extension pan 52. The tip 60 and the inner shaft 50 are thus joined at the tip attachment part 61. At the tip attachment part 61, therefore, the resin constituting the outer layer 58 described above and the resin constituting the tip 60 are mixed with each other and cover the braid 56 arranged on the inner layer 54. Therefore, the tip attachment part 61 has substantially the same outer diameter as the inner shaft 50. The inner diameter d1 of the tip attachment part 61 is substantially the same (about 0.41 mm) as the inner diameter of the inner shaft 50 described above.

In the present embodiment, the axial length L2 of the tip 60 is set at about 2.0 mm. The tip 60 substantially consists of the resin described above and does not include the braid 56. The outer diameter of the tip 60 progressively decreases toward the front end thereof compared to the outer diameter of the tip attachment part 61 (i.e. the outer diameter of the front end of the inner shaft 50). As shown in Fig. 4, the thickness of the rear end of the tip 60 is inwardly larger by t1 than the thickness T1 of the tip attachment part 61, that is, than the thickness of the front end of the inner shaft 50. With this configuration, the inner diameter d2 of the tip 60 is smaller than the inner diameter d1 of the tip attachment part 61. In the present embodiment, the inner diameter d2 of the tip 60 is about 0.38 mm.

The inner diameter d2 of the tip 60 is made small in this manner in order to make the tip 60 as thick as possible. The thick tip 60 alleviates the sudden stiffness change caused by the absence of the braid 56 at the boundary between the tip attachment part 61 and the tip 60. The diameter of the guidewire to be inserted into the inner shaft 50 is about 0.35 mm. In view of this, another reason for making the inner diameter of the tip 60 small is to minimize the difference between the diameter of the guidewire and the inner diameter d2 of the tip 60 (the gap between the guidewire and the inner surface of the tip 60). More specifically, the difference between the diameter of the guidewire and the inner diameter d2 of the tip 60 is about 5% to about 10% of the diameter of the guidewire. In this manner, in the balloon catheter 10, the difference between the diameter of the guidewire and the inner diameter of the tip 60 is small. With this configuration, the tip 60 is flexibly bent along the guidewire when the balloon catheter 10 passes through a tortuous vessel or a stenosis, or between stent struts, for example. As a result, a stepped portion is hardly generated between the front end of the tip 60 and the guidewire. Therefore, the balloon catheter 10 has an improved crossability in the vessel or the like.

The front end attachment part 22 of the balloon 20 is bonded to the front end of the extension part 52 of the inner shaft 50 and the rear end portion of the tip 60 by welding. The front end attachment part 22 also functions to improve the stiffness of the tip attachment part 61. This causes the stiffness change at the boundary between the tip attachment part 61 and the tip 60. Therefore, the front end attachment part 22 is fixed not only to the tip attachment part 61 but also to the tip 60 by welding. That is, upon fixing the front end attachment part 22 by welding, part of the molten resin constituting the front end attachment pant 22 flows toward the tip 60. As a result, the front end attachment part 22 is bonded smoothly in a tapered shape while extending from the tip attachment part 61 to the tip 60. In this manner, the sudden stiffness change is prevented.

A case will be described below in which the balloon catheter 10 according to the present embodiment having the above configuration is used in an operation for dilating a stenosis in a coronary artery in the heart.

A guidewire (not shown) is inserted in advance into the coronary artery of the heart where the stenosis to be treated is located. The balloon catheter 10 is inserted into the body along the guidewire. The rear end of the guidewire is inserted into the front-side guidewire port 69 of the tip 60 of the balloon catheter 10. The rear end of the guidewire passes through the guidewire lumen 51 in the inner shaft 50 and exits from the rear-side guidewire port 59.

An operator such as a physician pushes the balloon catheter 10 in the axial direction from the proximal side thereof in order to advance the balloon catheter 10 in the vessel along the guidewire. This pushing force is sequentially transmitted toward the distal side, that is, from the rear outer shaft portion 37 that is a metal tube, to the middle outer shaft portion 35 made of resin, the joint portion 33, and then to the front outer shaft portion 31.

Simultaneously, the pushing force is transmitted from the rear outer shaft portion 37 to the core wire 90 that is attached to the rear outer shaft portion 37. At this time, since the core wire 90 is joined to the inner shaft 50 and the front outer shaft portion 31 with the core fixing portion 38 of the joint portion 33, the pushing force is transmitted from the core wire 90 also to the inner shaft 50 and the front outer shaft portion 31. That is, the outer shaft 30 is pushed not only by the rear end thereof but also directly by the joint portion 33, which is an intermediate member, due to the core wire 90. As a result, the pushing force is effectively transmitted to the front end of the outer shaft 30.

Similarly, the core wire 90 pushes the joint portion 33 to which the rear end of the inner shaft 50 is attached. Therefore, the pushing force given from the proximal side is also transmitted to the inner shaft 50. The braid 56 is arranged on the inner shaft 50. This improves the pushability in the inner shaft 50. Furthermore, at the connecting portion between the front end of the inner shaft 50 and the tip 60, the inner diameter decreases from d1 to d2 due to the absence of the braid 56. This prevents the sudden stiffness change at the connecting portion. As a result, the pushing force is transmitted without being blocked at the boundary between the front end of the inner shaft 50 and the tip 60. Therefore, the pushing force can be transmitted not only to the outer shaft 30 but also to the inner shaft 50. Furthermore, the pushing force can be effectively transmitted from the inner shaft 50 to the tip 60 that is the front end of the balloon catheter 10.

When the balloon catheter 10 is to pass through a tortuous vessel or a stenosis, or between stent struts, the front end portion of the balloon catheter 10 such as the tip 60 may be stuck in, for example, the inner wall of the vessel or the stent strut to be blocked from passing through. In this case, if the operator rotates the rear outer shaft portion 37, the front outer shaft portion 31 joined thereto is rotated.

As in the case of the pushing force, the rotation of the rear outer shaft portion 37 is transmitted from the rear outer shaft portion 37 to the core wire 90. The rotation is then transmitted to the inner shaft 50 via the core fixing portion 38 of the joint portion 33. The braid 56 is arranged on the inner shaft 50. This improves the rotational force transmissibility of the inner shaft 50. Furthermore, at the connecting portion between the front end of the inner shaft 50 and the tip 60, the inner diameter decreases from d1 to d2 due to the absence of the braid 56. This prevents the sudden stiffness change at the connecting portion. As a result, the rotational force is transmitted without being blocked at the boundary between the front end of the inner shaft 50 and the tip 60. Therefore, the rotational force is transmitted to the front end of the tip 60. Therefore, even when the front end portion of the balloon catheter 10 such as the tip 60 is stuck in, for example, the inner wall of the vessel or the stent strut and the movement of the balloon catheter 10 is blocked, the rotation of the tip 60 can resolve the situation.

The inner diameter d2 of the tip 60, i.e., the diameter of the front-side guidewire port 69, is set smaller than the inner diameter d1 of the tip attachment part 61. The diameter of the front-side guidewire port 69 is minimized so as to approximate the outer diameter of the guidewire to be passed therethrough. With this configuration, the front end of the tip 60 is flexibly bent along the guidewire when the balloon catheter 10 passes through a tortuous vessel or a stenosis, or between stent struts, for example, along the guidewire. The stepped portion formed between the outer surface of the guidewire and the front end of the tip 60 is minimized. Therefore, the balloon catheter 10 can smoothly pass through the tortuous vessel or stenosis, or between the stent struts, for example.

In this manner, the operator advances the balloon catheter 10 in the vessel under radioscopy. The operator then positions, using the markers 70, the balloon 20 at the stenosis that is the target site. After that, the liquid for dilation, such as contrast agent or saline, is supplied from the indeflator (not shown) connected to the connector 80. The liquid for dilation flows into the rear inflation lumen 36d of the outer shaft 30, passes through the middle inflation lumen 36c and the communication hole 36b in the joint portion 33, and flows out of the front end of the front outer shaft portion 31, thereby dilating the balloon 20.

The procedure for dilating the stenosis is finished by dilation of the balloon 20. The operator then discharges the liquid for dilation out of the balloon 20 using the indeflator. After that, the balloon catheter 10 is pulled out of the body. Consequently, the entire operation is completed.

As described above, in the balloon catheter 10 according to the present embodiment, the pushing force and the rotational force applied by the operator are sequentially transmitted from the proximal side to the distal side of the outer shaft 30. Furthermore, the pushing force and the rotational force are transmitted from the middle portion of the outer shaft 30 via the core wire 90 and the joint portion 33.

The rear end of the inner shaft 50 and the joint portion 33 are joined. Therefore, the pushing force and the rotational force can be transmitted to the inner shaft 50 by the core wire 90. The braid 56 is arranged on the inner shaft 50. In addition, the inner diameter of the tip 60 attached to the front end of the inner shaft 50 is made small to reduce the difference in stiffness between the tip 60 and the inner shaft 50. Therefore, the pushing force and the rotational force are effectively transmitted to the front end of the tip 60.

Furthermore, the inner diameter d2 of the tip 60 is minimized so as to approximate the outer diameter of the guidewire to be passed therethrough. Therefore, the front end of the tip 60 can be prevented from coming into contact with and being stuck in the inner wall of the vessel or the stent strut at the time of passing through the tortuous vessel or between the stent struts.

In the embodiment described above, the rear end of the tip 60 positioned at the front end of the tip attachment part 61 and not including the braid 56 on the inner shaft 50 is thicker than the tip attachment part 61. With this configuration, the inner diameter decreases from d1 to d2 at the connecting portion between the front end of the inner shaft 50 and the tip 60. As described above, this configuration contributes to alleviating the stiffness change caused by the absence of the braid 56. Instead of the tip 60, however, a tip 160 shown in Fig. 7 may be used considering the hardness or the like of the resin constituting the tip. A bore of the tip 160 extending over the length L3 (the rear end portion of the tip 160) from the front end of the tip attachment part 61 (the rear end of the tip 160) toward the front side in the axial direction has the same inner diameter as the inner shaft 50, that is, the inner diameter d1. The inner diameter of the other portion of the tip 160 (on the front side of the aforementioned portion of the tip 160) decreases to d2.

In this case, the outer diameter of the tip 160 decreases toward the front end thereof also at the rear end portion of the tip 160. As shown in Fig. 7, therefore, the maximum thickness of the tip 160 is smaller by dt than that of the tip 60 shown in Fig. 4. However, the increment in thickness t1 is set larger than the decrement dt. Therefore, the influence of the decrement dt on the effect of the present embodiment described above is minimal (the decrement dt is shown in an exaggerated manner in Fig. 7).

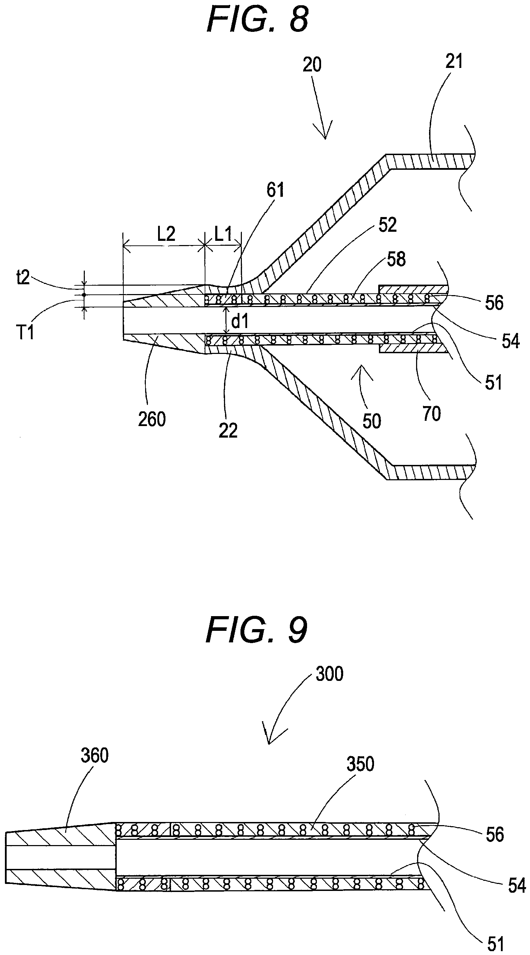

In the embodiment described above, the thickness of the tip 60 is increased by decreasing the inner diameter d2 of the tip 60. This configuration reduces the difference in stiffness between the tip attachment part 61 with the braid 56 provided thereon and the tip 60 without the braid 56. As in the case of a tip 260 shown in Fig. 8, however, the thickness thereof may be increased by t2 by increasing the outer diameter of the tip 260. Also in this case, it is possible to reduce the difference in stiffness between the tip attachment part 61 with the braid 56 provided thereon and the tip 260 without the braid 56. In such a case, it is somewhat difficult to decrease the gap between the tip 260 and the guidewire to be inserted therethrough. However, this embodiment has a similar effect to the embodiment described above in transmitting the pushing force and the rotational force to the front end of the tip 260.

In the embodiments described above, the rear-side guidewire port 59 is opened at the side surface of the outer shaft 30. Therefore, the balloon catheter 10 is a so-called rapid exchange type balloon catheter with the short guidewire lumen 51. However, the inner shaft 50 may be arranged to reach the rear end of the balloon catheter 10. In this case, the balloon catheter 10 is a so-called over-the-wire balloon catheter. The over-the-wire balloon catheter has an inner shaft extending to the rear end of an outer shaft. This makes it easy to transmit the pushing force and the rotational force. Therefore, the core wire may not be provided.

In the examples described in the above embodiments, the present invention is applied to the balloon catheter. However, the present invention can also be applied to a catheter other than the balloon catheter. For example, a micro catheter 300 is shown in Fig. 9. In the micro catheter 300, a tip 360 similar to the tip 60 described above is provided at the front end of a shaft 350 having a similar configuration to the inner shaft 50 in the embodiments described above.

Note that in the embodiments shown in Figs. 7, 8, and 9, substantially the same constituent elements as those of the embodiment shown in Figs. 1 to 6 are denoted with the same reference signs.

In the embodiments described above, the braid 56 is used as the reinforcing member. Alternatively, however, a coiled body formed by winding one strand, or a stranded wire coil formed by twisting a plurality of strands may be used as the reinforcing member. Note that the braid or the stranded wire coil is more effective than the coiled body formed of one strand in transmitting the pushing force and the rotational force.

In the embodiments described above, the catheter is used for the treatment of a vessel in the heart. However, the catheter according to the present invention can be used for various operations such as an operation for dilating a vessel in the lower limb or a shunt for dialysis.

While the invention has been shown and described in detail, the foregoing description is in all aspects illustrative and not restrictive. It is therefore understood that numerous modifications and variations can be devised without departing from the spirit and scope of the invention as defined by the appended claims.

## Claims

1. A catheter (10) comprising:
a shaft (50) including
a tubular inner layer (54),
a reinforcing member (56) arranged on an outer circumferential surface of the inner layer (54), and
an outer layer (58) covering the reinforcing member (56);
**characterized by**:
a tubular tip (60) made of resin and attached to a front end of the shaft (50), where the tip (60) has a portion thicker than the front end of the shaft (50), and the tip (60) has an outer diameter decreasing toward a front end of the tip (60).

2. The catheter (10) according to claim 1, wherein the tip (60) has an inner diameter (d2) smaller than an inner diameter (d1) of the shaft (50) to make the portion of the tip (60) thicker than the front end of the shaft (50).

3. The catheter (10) according to claim 1 or 2, wherein the portion of the tip (60) thicker than the front end of the shaft (50) is positioned at a rear end of the tip (60).

4. The catheter (10) according to any one of claims 1 to 3, further comprising:
a balloon (20); and
a tubular outer shaft (30) to which at least part of the balloon (20) is joined,
wherein the shaft (50) is inserted into the outer shaft (30).

## Amended claims

### Amended claims in accordance with Rule 137(2) EPC.

**1.** A balloon catheter (10) comprising:
a shaft (50) including
a tubular inner layer (54),
a reinforcing member (56) arranged on an outer circumferential surface of the inner layer (54), and
an outer layer (58) covering the reinforcing member (56); **characterized by**:
a tubular tip (60) made of resin and attached to a front end of the shaft (50), where the tip (60) has a portion thicker than the front end of the shaft (50), and the tip (60) has an outer diameter decreasing toward a front end of the tip (60).

**2.** The balloon catheter (10) according to claim 1, wherein the tip (60) has an inner diameter (d2) smaller than an inner diameter (d1) of the shaft (50) to make the portion of the tip (60) thicker than the front end of the shaft (50).

**3.** The balloon catheter (10) according to claim 1 or 2, wherein the portion of the tip (60) thicker than the front end of the shaft (50) is positioned at a rear end of the tip (60).

**4.** The balloon catheter (10) according to any one of claims 1 to 3, further comprising:
a balloon (20); and
a tubular outer shaft (30) to which at least part of the balloon (20) is joined,
wherein the shaft (50) is inserted into the outer shaft (30).

**5.** The balloon catheter (10) according to claim 1, wherein a front end attachment part (22) of the balloon is bonded to a front end of an extension part (52) of the shaft (50) and a rear end portion of the tip (60).

**6.** The balloon catheter (10) according to claim 5, wherein the front attachment part (22) has a tapered shape decreasing from the shaft (50) toward the tubular tip (60).

**7.** The balloon catheter (10) according to claim 5 or 6, wherein an outer diameter of the thick portion of the tip (60) is larger than that of the front end of the shaft (50), and the front end attachment part (22) is in contact with the thick portion of the tip (60) and the front end of the shaft (50).
